# EUROPEAN PATENT APPLICATION

(11) **EP 1 967 216 A1**
(43) Date of publication of application: **10.09.2008**
(21) Application number: 08397503.7
(22) Date of filing: 23.01.2008
(51) Int. Cl.: A61L 9/12, A61L 9/14

(54) **Air freshener**

(30) Priority: 24.01.2007 FI 20070031 U
(71) Applicant: Metsä Tissue Oyj, 02100 ESPOO (FI)
(72) Inventor: Achton, Kim, 4632 Bjaeverskov (DK); Mattila, Iiro, 35300 Orivesi (FI)
(74) Representative: Haimelin, Jukka Ilmari

(57) **Abstract**

An air freshener comprising a changeable carrier (1) for the air freshening medium, electrically operated means for dosing the air freshening medium to the surrounding air, and a refillable electric current source (3) for the dosing means. The operation site of the electric current source is included in the carrier of the air freshening medium.

## Description

The present invention relates to an air freshener comprising a changeable carrier for the air freshening medium and electric means for dosing the air freshening medium to the surrounding air. The dosing means for the air freshening medium can be for example a fan. By means of the fan, air flow through the device and spreading of the flow to the surrounding space can be provided. The air flow passing through the device takes perfume (air freshening medium) located in a carrier suitable for the device, and carries it along to the surrounding of the device. The fan is driven by a motor being powered by an electric current source located in the device. The current source is of the type storing the electric energy, in praxis a battery or an accumulator that must be replaced (refilled) when it runs out of stored electric energy. Correspondingly, the air freshening medium is refilled when it is running out in the carrier or has lost its effect.

The air freshening medium can be in the device as a gel, whereby the air frehening medium moves from the gel to the air being brought to flow over it by means of the fan. There are also devices known in the art, wherein the air freshening medium is sprayed with suitable devices to the surrounding air or to the air led through the device. For practical reasons, the required electricity is taken from a battery or an accumulator.

One problem with the devices known in the art has been the work required by the operation, requiring, on one hand the refilling of the air freshening medium and on the other hand the refilling of the current source. These operations are usually needed at different time intervals. The current source may be exhausted, even though the air freshening medium is still in effective condition, and correspondingly, the running out of the air freshening medium does not necessarily mean the need to replace of the current source.

The work needed for the service of the devices has been significantly decreased by means of an air freshener according to the present invention. At the same time, the air quality in the public sanitary facilities can be improved, and the maintaining of the air quality will be secured. The air freshener in accordance with the invention comprises, as known in the art, a changeable carrier for the air freshening medium, electrically operated means for dosing the air freshening medium to the surrounding air, and a refillable electric power source for the dosing means. The invention is characterized in that the operating site of the electric current source is included in the carrier of the air freshening medium. With this constructional solution it can be secured that both the replacing of the air freshening means cartridge and the replacing of the current source will be performed, and can be performed as a single operation.

The constructional solution also secures the refilling of the air freshening medium on time, in case a monitoring mechanism of the operation is included in the device. This mechanism can be implemented reliably so that it controls the charge level of the current source. When the charge levels goes below the detection limit value, the device gives an indication and the current source and the refilling of the air freshening medium are taken care of on time, presumed naturally that the amount of the air freshening medium has been dimensioned according to the capacity of the current source.

The operation of the control device can also be linked with the means for measuring time. The time measuring means is reset when the cartridge including the air freshening medium and the current source have been replaced, and will give a new reminder again when the deadline based on the consumings is approaching.

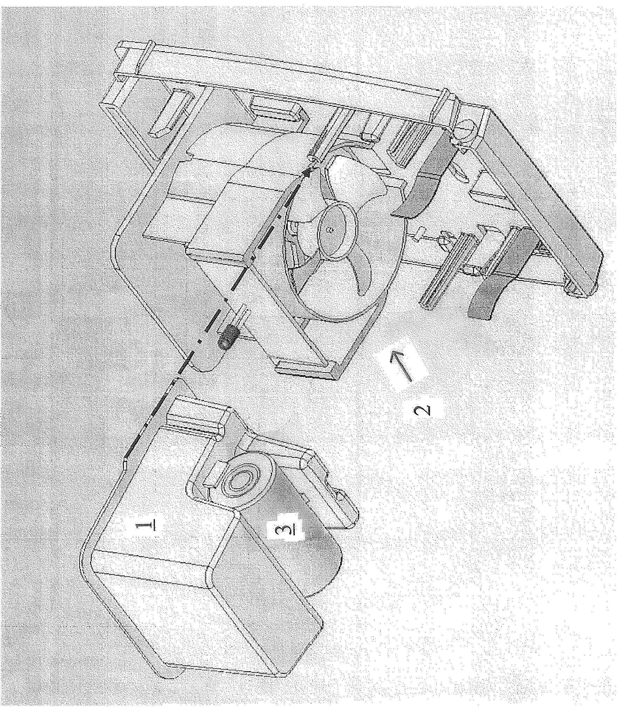

The invention will be described in more detail with reference to the enclosed drawing showing a figure of one embodiment of the present invention.

The figure shows a wall-mounted air freshener for example for public sanitary facilities. The basic parts of the device are the fan 2 providing the air flow through the device. The fan is powered by a battery 3. The air freshening medium is located in a bowl-like vessel 1, for example as a gel. The air flow effected by the fan is led over the upper surface of the vessel 1, said vessel 1 either being covered with a porous or perforated lid or being alternatively open.

The operating position of the current source, i.e. battery 3 is located in connection with the vessel 1, preferably with a fixed attachement thereto. Thereby the vessel and the operating site fixed thereto form a unit having a cartridge construction, that can be located in the fasteners and fittings so that the electric connection for the fan can be provided. Additionally, the apparatus can include the above mentioned control means of the operation, not shown in the figure, having the construction and operation known by those skilled in the art.

## Claims

1. An air freshener comprising a changeable carrier (1) for the air freshening medium, electrically operated means for dosing the air freshening medium to the surrounding air, and a refillable electric current source (3) for the dosing means, **characterized in that** the operation site of the electric current source is included in the carrier of the air freshening medium.

2. An air freshener in accordance with Claim 1, **characterized in that** the dosing means is a fan (2) for providing an air flow being in contact with the air freshening medium.

3. An air freshener in accordance with Claim 2, **characterized in that** the air freshening medium is included in a gel located in a vessel.

4. An air freshener in accordance with Claim 1, **characterized in that** the dosing means is a means spraying the air freshening medium.

5. An air freshener in accordance with any of the preceding claim, **characterized in that** it is equipped with an indicator of the charge level of the electric current source.

6. An air freshener in accordance with Claim 5, **characterized in that** the charge level indicator is a time counter.
